# EUROPEAN PATENT SPECIFICATION

(11) **EP 1 180 348 B1**
(45) Date of publication and mention of the grant of the patent: **03.12.2008**
(21) Application number: 01306782.2
(22) Date of filing: 08.08.2001
(51) Int. Cl.: A61B 17/70

(54) **Orthopaedic rod/plate locking mechanism**
Orthopädische Stange / Verriegelungsmechanismus für eine Platte
Tige orthopédique / mécanisme de verrouillage de plaque

(30) Priority: 08.08.2000 US 633057; 17.04.2001 US 836445
(43) Date of publication of application: 20.02.2002
(60) Divisional application: 07075744.8
(73) Proprietor: DePuy Spine, Inc., Raynham, MA 02767-0350 (US)
(72) Inventor: Harkey, Louis, Mississippi 39157 (US); Currier, Bradford, Minnesota 55902 (US); Selvitelli, David M., Massachusetts 02482 (US); Reynolds, Martin A., Massachusetts 02035 (US); Doherty, Thomas V., Massachusetts 02035 (US)
(74) Representative: Mercer, Christopher Paul

(56) References cited:
- WO-A-95/31147
- DE-A- 19 636 309
- US-A- 5 558 674
- US-A- 5 928 233
- US-A- 6 086 590

## Description

### REFERENCE TO RELATED APPLICATIONS

### BACKGROUND

The present invention relates to fixation devices used in orthopaedic surgery and particularly to devices used for the reduction of fractures or the positioning of bones by means of a plate attached to a bone or bone fragment in one region and secured to a rod which attaches to a cable, wire, plate or screw fastened in another region. The rod thus attaches between two bone regions for effecting stabilization, positioning, reduction or fixation of the bones.

A number of such mechanisms are known, among which should be mentioned the Harms T-plate which employs a split or slotted bolt, the head of which slides in a slot of a plate that is attached to a bone or bone fragment. The plate accepts the slotted bolt from the bottom and has several channels or grooves extending in different directions in the plate to allow positioning and alignment of the bolt along any one of the distinct channels. In use, a connecting rod fits through the slotted bolt and is captured by a nut which, when tightened, locks the bolt in its position in the channel, and secures the rod in the slot. In general, the system employs a slotted bolt with a square flange at its base so that each of the channels defines orientation of the rod-receiving slot of the outwardly protruding portion of the bolt. The plate thus provides a range of linear positions along several discrete lines, each at a fixed angular orientation, for the rod anchor point. See also US5928233 for an illustration of a slotted bolt.

In addition to such plates, for posterior cervical fixation there also exist a number of eye screws that screw directly into the bone at a single fixed position. In these screws, the eye structure generally is an open slot or other rod-receiving open form adapted to receive the rod therein before being closed by a cap. The cap may be a conventional threaded locking nut, or in some constructions may be a dovetailed cap segment which slides in and wedges against the rod to secure the rod while closing the receiving slot at its open end. Such eye screws may also, in some constructions, be employed to secure a plate to the bone in addition to gripping the stabilization rod. When so used, the plate serves to strengthen the attachment and distribute the stresses coupled by the anchoring screw.

One variant of a rod-receiving fixation screw is the Moss-Miami polyaxial screw, as shown in United States Patent No. 5,672,176. In that device, the screw has a spherical head. A slotted rod-holding cap structure having a conically tapering inner surface fits about the outside of the spherical head in the manner of a ball and socket joint. The rod-holding cap structure is internally threaded and is provided with a number of shaped packing or pressure-bearing inserts with an overall structure that tightens about the spherical screw head as the cap is drawn upward forcing the head down the cone angle. The cap may be rotated before tightening on the spherical head, so this clamping connection allows the rod-holding member to be bolted down and fixed with its slot oriented at an arbitrary angle in rotation about the axis of the screw. The rod fits through the slot in the holding cap structure and is secured by tightening a bolt into the threaded cap. The unit comes as a pre-assembled device with the packing or pressure-bearing members positioned internally about the ball end of the screw and held by swaging part way up the cap. Tightening of the cap against the rod then draws the conical outer holding body upward against the ball, fixing the slot orientation with the rod in position.

When the underlying bone has sufficient integrity, such individual eye screws offer great flexibility in rod orientation in one plane. Also, when a bone plate secured by multiple screws is necessary, the Harms plate offers a range of clamping point translational positions with a discrete set of angular orientations for connecting a cable, fixation rod or reduction rod. However, each of these systems has its own limitations as to convenience, or as to the range of position or orientation, or to the degree of loading that it may accommodate. In addition, the various plates, and screw heads or cap structures may present bulky or irregular profiles that pose obstacles to effective anchoring or surgical closure in some locations. Other fixation devices are disclosed in US 5558674, US5928233, WO95/31147 and DE 19636309. It would be desirable to provide a bone plate and rod junction system of adjustable angulation.

It would also be desirable to provide a multi-axis rod connection that is freely positionable along a slotted plate.

It would also be desirable to provide a multi-axis rod connection in which the components are pre-assembled to be installed as a unit during surgery.

It would also be desirable to provide an improved anchor plate structure.

It would also be desirable to provide improved methods of spinal fixation embodying mechanical anchor, rod and cabling units effective to fix and secure the spine.

The features of the present invention that are known from WO95/31147 have been placed in the preamble of claim 1 appended hereto.

### SUMMARY OF THE INVENTION

The present invention is defined by claim 1.

Preferred features are disclosed in the dependent claims.

### BRIEF DESCRIPTION OF THE DRAWINGS

These and other features of the invention will be understood from the description herein, taken together with drawings of illustrative embodiments, wherein
Figure 1 shows an exploded perspective view of the rod junction system of the present invention;
Figure 2 shows elements of the embodiment of Figure 1 seated on a plate;
Figure 3 shows a cross-sectional view through the system of Figure 1;
Figure 4 illustrates an offset tab embodiment;
Figure 5 illustrates another embodiment of an anchor bolt and support washer in accordance with the invention;
Figure 5A illustrates another embodiment of a support washer;
Figure 6 shows another embodiment of an anchor bolt and support washer in accordance with the present invention;
Figures 7-7B illustrate another embodiment of a support washer for the practice of the present invention;
Figures 8-8A illustrate an anchor bolt of the present invention for use with the support washer embodiment of Figures 7-7B;
Figures 9-9B illustrate another embodiment of a support washer assembly of the present invention;
Figures 10 and 10A illustrate an anchor bolt embodiment for use with the washer assembly of Figures 9-9B;
Figure 11 shows one occipital fixation plate for use in an assembly of the present invention;
Figures 11A-11B show another occipital fixation plate;
Figures 12A-12C illustrate another occipital fixation plate;
Figures 12D and 12E illustrate positioning of the occipital plates of Figures 11 and 12A-C, respectively;
Figure 12F illustrates cable layout according to one embodiment; and
Figures 13A and 13C illustrate a cable connector and its use in a method of spinal fixation according to a system of the invention.

### DETAILED DESCRIPTION

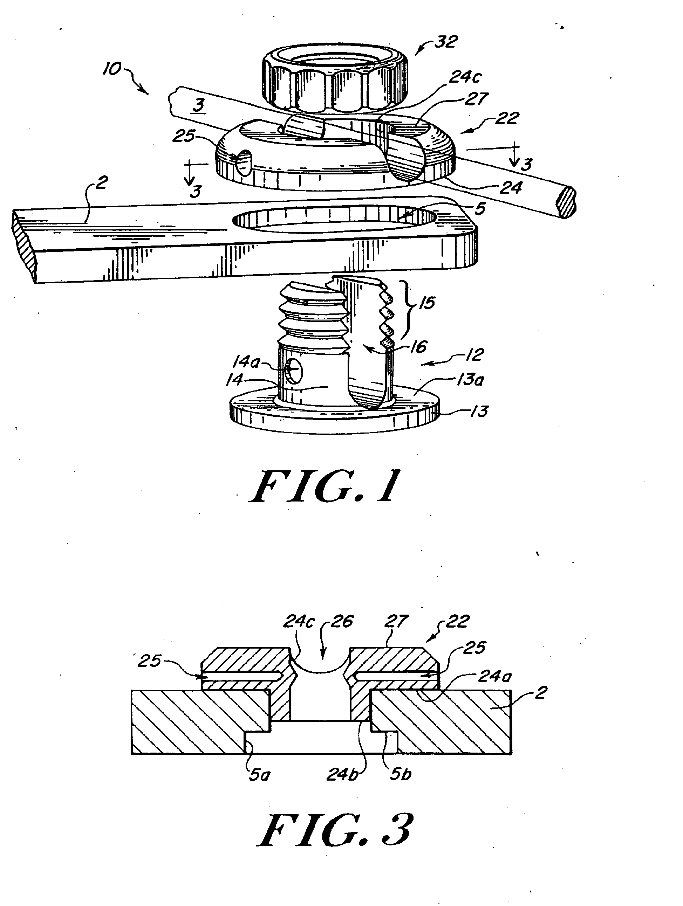

Figure 1 shows an exploded perspective view of one embodiment of the rod junction system 10 of the present invention. As shown, in this embodiment, the invention is comprised of a plate 2 through which a slotted bolt 12 fits, with a rod-contacting support platform 22 or support collar fitted over the bolt 12, and a nut or cap 32 fitted onto the end of the bolt to secure the rod. Rod 3 is shown in phantom for purposes of illustration. The aperture 5 in the plate 2 may be a simple round hole, an elongated but closed-ended slot as illustrated, or an open-ended slot of the type known in the prior art that allows the bolt 12 to be moved to various positions along the plate length. Furthermore, the plate may take any of a number of configurations of the various shapes commonly used in orthopaedic fixation. That is, the plate may be generally planar, and shaped like a T or a Y or a V.

It may also be bent or curved out-of-plane to fit a curved bone surface, and may be installed in various orientations. In one embodiment, the assembly is used with a plate 2 that has the contour of an occipital bone fixation plate, and the bolts 12 are positioned at both sides of a center line for securing two fixation rods extending in parallel to the spine. Exemplary T-shaped, Y-shaped and inverted-Y plates and methods of installation are described further below.

As shown in Figure 3, the hole or slot 5 of plate 2 is counter-bored or milled to a larger opening 5a, so that the base 13 of the slotted bolt fits up in and is recessed from the level of the bottom surface of the plate 2, against a step or thrust face 5b. Thus, the opening 5 may be a counter-bored hole or elongated hole, or one of several step-walled slots. As further shown in Figure 1, the bolt 12 includes a shank portion 14 extending from the base 13, and a threaded shaft portion 15 extending above the shank. A U-shaped slot 16 runs the length of the threaded shaft 15 and preferably extends through at least a portion of the shank 14. Furthermore the base 13 in the illustrated embodiment is generally disk-shaped or radially symmetric in that it allows the bolt 12 to rotate freely in the hole or slot of plate 2 through one full revolution. The base 13 is considerably wider than the hole 5, so the bolt 12 cannot be pulled through the plate.

On the other side of the plate 2, the support platform 22 or thrust collar fits around the threaded shaft of the bolt and has a shallow yoke or transverse groove 26 (Figure 3) formed in its top surface 27. In use, groove 26 aligns with the slot 16 of the bolt 12, and forms a bearing or seating surface on which the rod 3 rests. The support platform 22 is formed as an annular washer that functions as a support anvil for the rod 3 and also as a collar or sleeve about the bolt 12. It thus operates together with the bolt to reinforce the apertured plate 2 and effectively sandwich the plate between the base 13 of the bolt and the platform 22 itself.

As further shown in the vertical sectional view of Figure 3 taken in a diametral plane of the bolt transverse to the direction of the slot, for this purpose the platform 22 has a lower surface 24 comprised of a washer-like body portion with an outer peripheral surface in a band 24a that rests on the top of the plate 2, and an inner annular portion 24b which extends into the plate opening 5 and forms a collar or reinforcement sleeve within the opening 5 of the plate 2. In this embodiment, a pair of radially directed holes 25, of which one is visible in Figure 1, extend inward from the circumferentially outer wall of the platform 22 to a depth close to its radially inner surface 24c. As best seen in Figure 3, the holes 25 serve as access holes to permit swaging the remaining thin-walled inner collar portion of the support platform member 22 to the bolt 12 while the plate 2 is captured between the bolt base 13 and the platform 22, so that the entire assembly forms a single unit loosely held together and freely movable without danger of losing the parts during handling prior to installation.

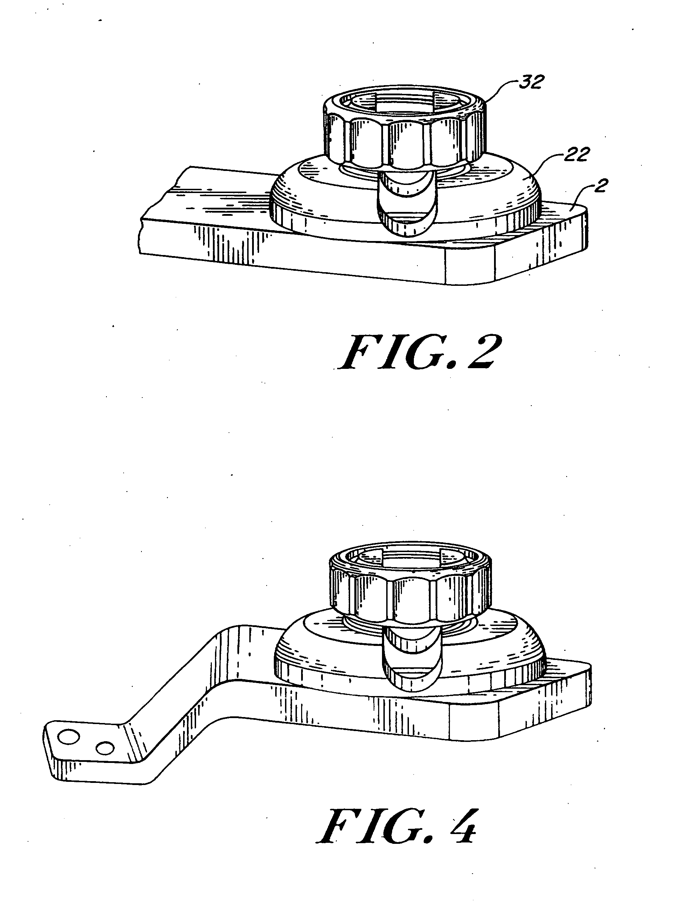

In the embodiment illustrated in Figures 1 and 2, the components are dimensioned so that the platform 22 is swaged to the shank 14 of the bolt below the threaded region 15, and at positions transverse to the axis of the slot 26. For this purpose cross-holes or recesses 14a are preferably drilled or otherwise formed in the shank at positions corresponding to the deformed swaging of the inner wall. Like the base 13 of the bolt 12, the platform 22 extends radially outward beyond the aperture 5 so that when it is swaged together with the bolt, the plate 2 is captured therebetween while the bolt and support platform assembly 12, 22 may rotate freely together as a unit to any angular position in a plane transverse to the axis of the bolt shaft. As shown, the unthreaded shank portion 14 of the bolt extends for a length roughly equal to the thickness of plate 2 and platform 22, and the threaded portion of the shaft 15 extends for a sufficient further length to allow the nut 32 to clamp the rod and bolt assembly together.

Figure 2 shows the platform 22 resting in position on the plate 2, with the bolt 12 omitted for clarity. As shown, the thickness of the support platform 22 constitutes a substantial structural reinforcement of the plate anchoring area. The groove in the platform member may taper inward slightly toward its base so that when the rod 3 is inserted in the slot and clamped downwardly, it wedges or fits closely against the sides of the supporting groove, adding rigidity to the overall system. The overall length of the bolt 12 is preferably such that the threaded portion extends only slightly, for example, less than a centimeter, above the top of the platform 22 to accommodate the nut 32, and the nut thus resides with a low profile over the rod and plate once it has been installed.

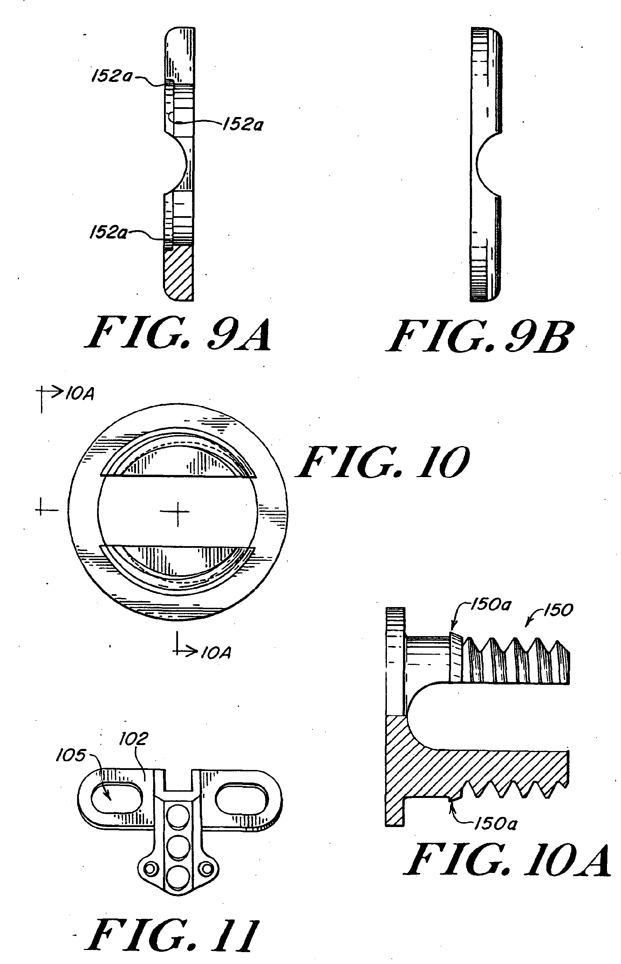

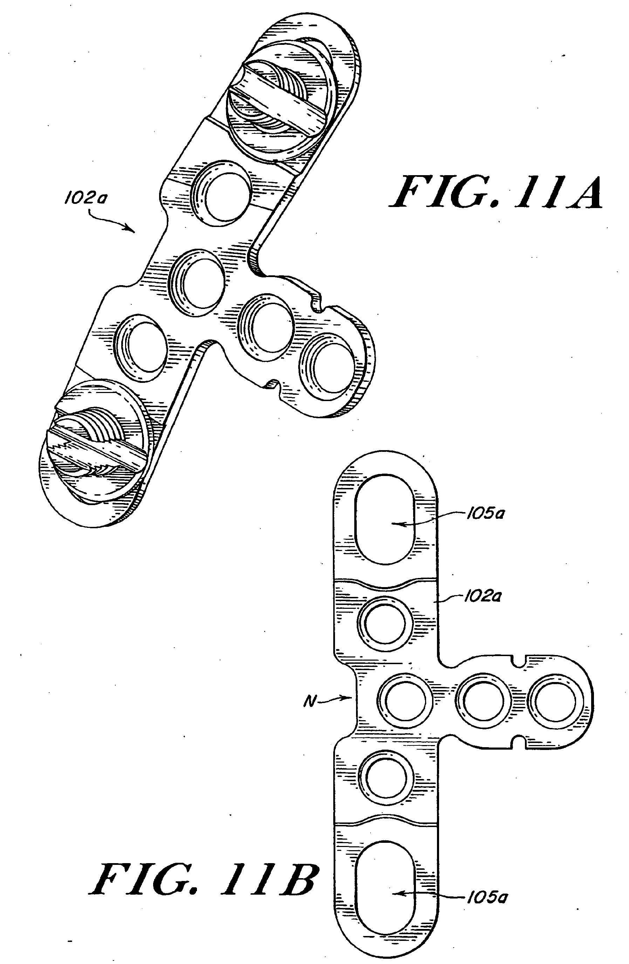

Figure 11 shows by way of example one occipital fixation plate 102 in accordance with the present invention. Plate 102 includes two apertures 105, each of which accommodates a slotted bolt assembly, which may be any of the embodiments shown in the figures herein or their equivalents. As shown, for the occipital plate 102 the apertures 105 are elongated in the lateral direction to allow adjustment of bolt position in a side-to-side direction before tightening down to secure a rod, cable or other fixation linkage.

Figures 11A-11B show a top perspective view and a top plan view, respectively, of another occipital fixation plate 102a in accordance with the present invention. Plate 102a, like plate 102, is T-shaped and includes two apertures 105a, one on each arm of the T, to accommodate slotted bolt anchor assemblies as discussed above for the apertures 105. The apertures 105a, elongated in the lateral direction to allow adjustment of bolt position in a side-to-side direction before tightening down to secure a rod, cable or other fixation linkage. Three chamfered holes extend along the midline for bone screws, and one additional bone screw opening is provided on each side arm to firmly fasten the plate against the occiput. The arms of the plate may curve, or extend at a slight dihedral angle to the central line of the T to conform to the skull.

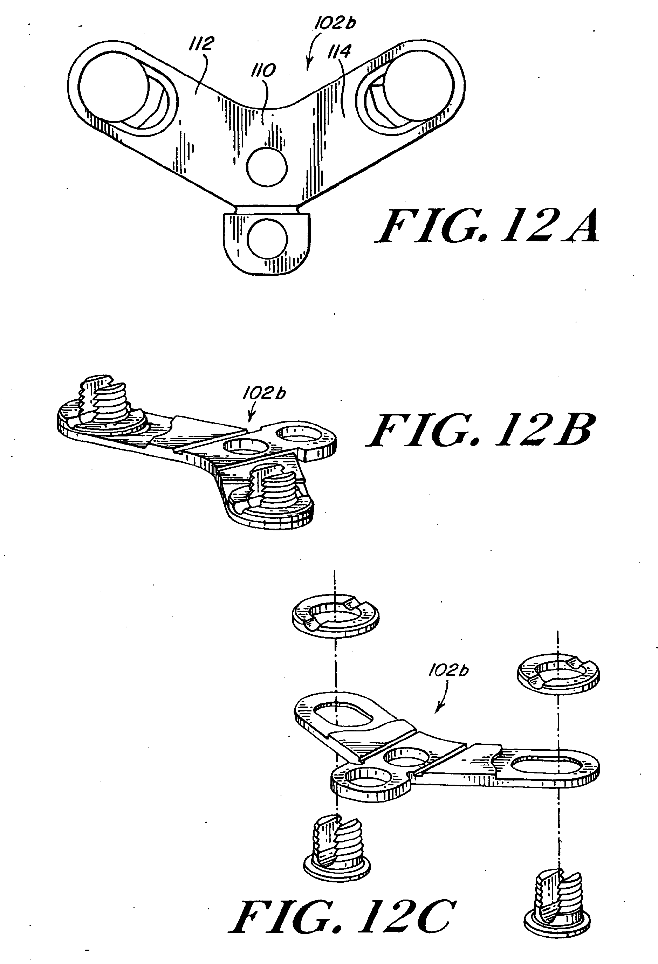

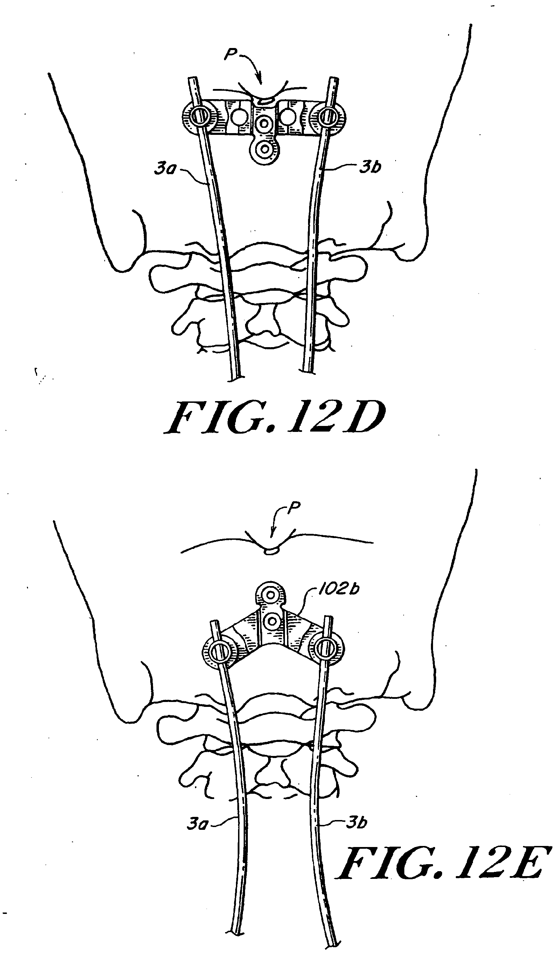

Figure 12D illustrates positioning of the T plate in use. The midline notch N (Figure 11B) on the superior aspect of the plate is aligned with and positioned below the external occipital protuberance P of the skull, and aligned along the superior nuchal line. This positions the openings for the bone screws along the thickened internal central line of the occiput, assuring a stronger anchor.

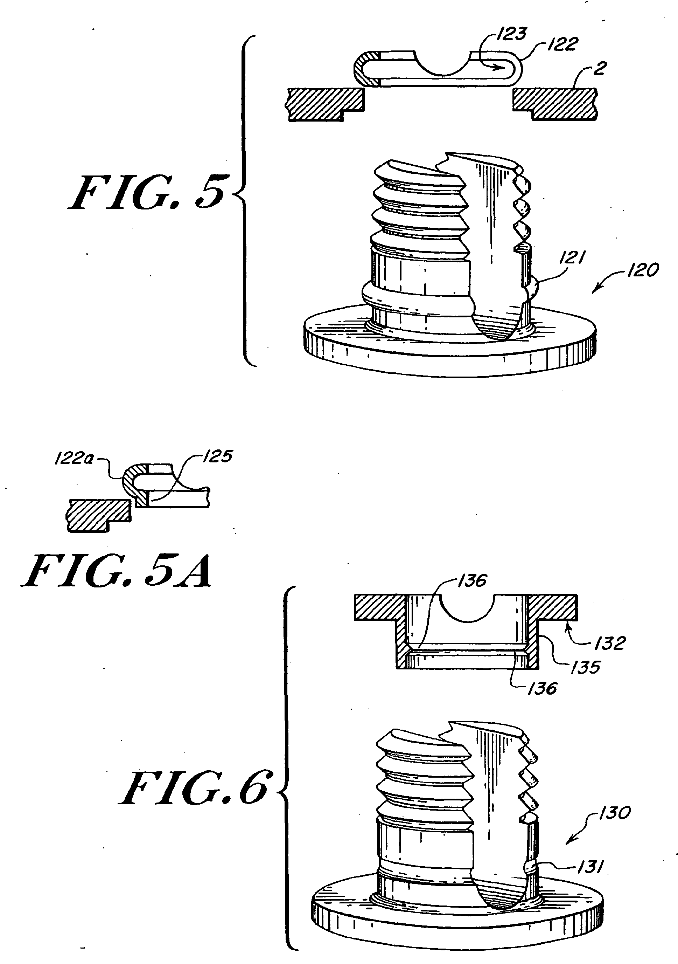

The construction described above, wherein a bolt and swaged support washer assembly captures the plate, thus forms a conveniently installed one-piece plate and anchor assembly of versatile angulation for securing a fixation rod. In addition, the invention contemplates other embodiments. Figure 5 illustrates one such embodiment, wherein an anchor bolt 120 is formed with a protruding circumferential ridge 121 positioned to capture the support washer 122 in a unitary assembly. The support washer 122 in this case has a corresponding groove or recess 123 into which the ridge fits to lock the washer onto the bolt and capture the plate 2. This grooved support washer may have a somewhat lower profile than that illustrated in Figures 1-3. Further, it need not have a structural portion corresponding to the dependent sleeve portion 24b of the first-described embodiment, although such a portion may be provided as a centering collar or sleeve 125 shown in Figure 5A for the support washer 122a.

Figure 6 shows another embodiment of an anchor bolt and support washer 130, 132 in accordance with the present invention. As with the other embodiments, the two pieces fasten together to capture the bone plate and provide a nonseparating and unitary assembly that may be freely manipulated during installation, and that allows the slotted bolt to be adjusted in angle, and in some embodiments also in linear position, prior to tightening down of the bolt over the rod or cable linkage assembly. In the embodiment of Figure 6, the bolt 130 possesses a recessed circumferential groove 131, and the support washer 132 has a corresponding portion with a protruding ridge 136 that snaps into the groove 131 to retain the two parts together. Ridge 136 need not be a continuous ridge, but may consist of one or a small number of slight bumps or protrusions which are sized to allow movement into the groove 131 with a slight pressure, and without shearing or cracking of the contacting parts. When bumps rather than a ridge are provided, the groove 131 may also be replaced by a few discrete indentations, in which case the indentations and bumps may further be positioned at angles selected to align the bolt slot with the rod support groove. A dependent collar portion 135 allows the mating ridge 136 or protruding bumps to be positioned quite low on the bolt shank, so that the device has an overall profile that extends only slightly above the plate to which it is mounted.

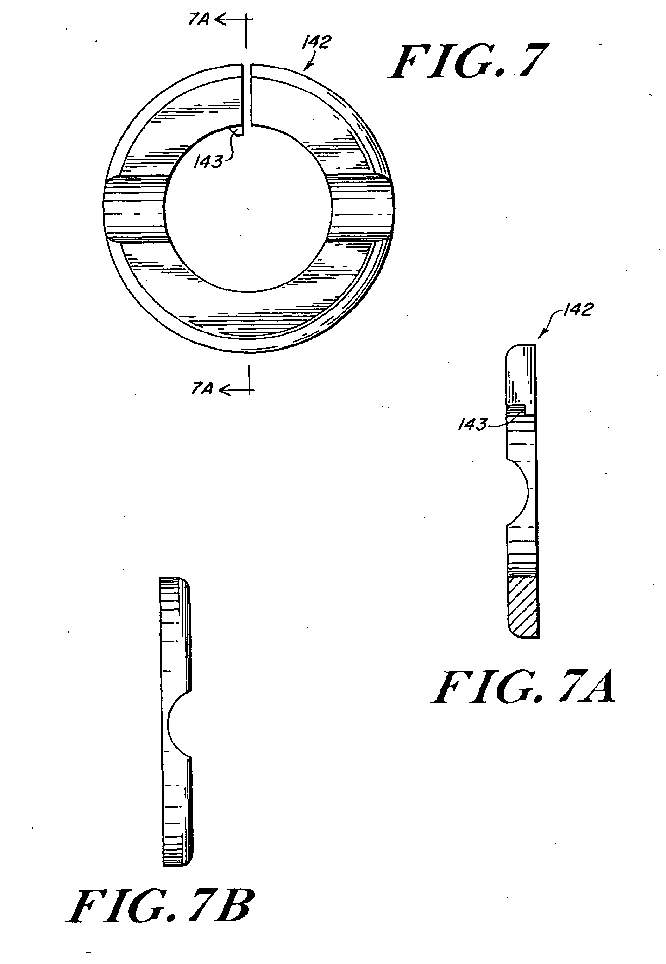

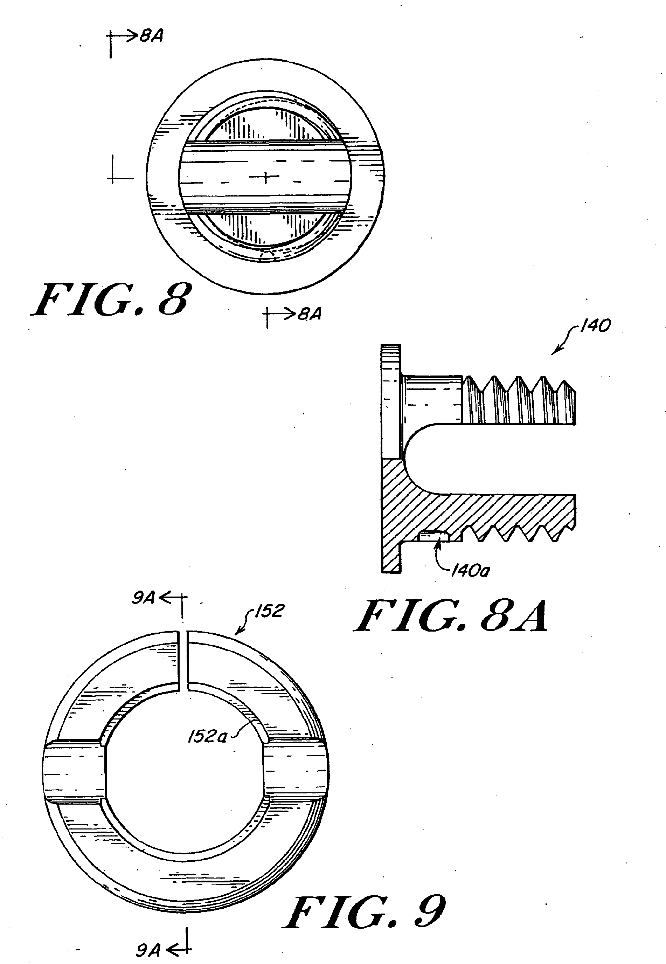

Figures 7-7B and 8-8A illustrate another embodiment of an orthopaedic anchor bolt 140 and support washer 142 for the practice of the present invention. As with the other embodiments, the washer has a transverse groove or seating surface for supporting the fixation rod or cable, and has a generally flat washer-like body that rests against the underlying bone plate. In the embodiment of Figure 7 the washer 142 is preferably a split washer having a radially extending gap 142a that allows the washer to flex open and be placed over the bolt 140 such that a tooth 143, which projects radially inward, engages a corresponding recess 140a in that bolt. Figure 7 shows the washer in a top plan view, while Figure 7A illustrates a vertical section taken along a diametral plane and through the washer gap 142a. Figure 7B shows a side plan view, illustrating the flat upper and lower surfaces of the washer. Figure 8 is an end view of the bolt, 140, taken along the axial direction from above the slot of the bolt, with a dotted section line illustrated by A-A showing the direction of the partial cut away sectional view of Figure 8A. As shown in Figure 8A a recess 140a approximately one millimeter deep is provided in the bolt shank to capture the protruding tooth 143 of the support washer.

Figures 9-9B, 10 and 10A illustrate another embodiment of an anchor bolt and washer assembly 150, 152 of the present invention. These views correspond to those of Figures 7-7B, 8 and 8A, with similar features appearing similarly in the two figures. In this embodiment, however, bolt 150 is provided with a catch or radially-protruding and sharply angled edge 150a extending radially outward near the top of the shank portion of the bolt. The catch 150a catches the radially inward edge 152a of an upper surface of the support washer 152. For this purpose the radially inner region of the support surface is recessed slightly in the axial direction, so that the fastening edge 152a of the support washer 152 is lowered, at the level of the shank of the bolt 150. The entire assembly therefore has a low profile. The recessed inner step also protects the fastening edge from becoming nicked, rounded or otherwise impaired if a bulk finishing process such as tumbling, is employed to deburr or finish the support washer.

Thus, the anchor assembly may be implemented with a number of different possible washer or collar-like support elements to capture the plate and provide a freely oriented anchor bolt assembly. The plate itself may take varied forms, which do not necessarily form part of the invention, including individual vertebra plates, hooks or offset elements, or may be shaped like an occipital T-plate, forming an assembly with one or more anchor bolts.

It will be understood that in providing fixation rods to effect spinal alignment, fixation and fusion, the rods themselves and the rod-securing bolt assemblies 12, 22, 32 protrude upward from the bone, and closure of the surgical wound requires that surrounding soft tissue be closed and sutured over these projecting assemblies. For elderly and other patients whose tissue lacks sufficient elasticity, this may be difficult to achieve.

This problem is addressed in a further aspect of the invention by providing a Y-shaped occipital fixation plate that fastens in an inverted orientation and positions anchor bolts 12 lower down the skull, e.g., below the inferior nuchal line and in a position where the soft tissue is substantially thicker and better able to stretch and cover the mechanical components of the implanted assembly. Figures 12A-12C illustrate such a Y-shaped occipital fixation plate 102b.

As shown in Figures 12A-12C, the plate 102b has first and second branch arms 112 114 of the Y that extend to both sides of a central portion 110, and each arm is adapted, as described above, to receive a rod anchor bolt. In one preferred construction and method of use, the central portion 110 of the Y-plate forms a short central trunk, and the plate is located and secured in an inverted orientation as shown in Figure 12E, with the Y-arms extending downwardly and out from the trunk, in an inverted Y position. The central trunk extends upwardly along the midline toward the exterior occipital protuberance P. The exterior occipital protuberance and the posterior margin of the foramen magnum may be used as a guide to the midline of the occiput, and the inverted Y-plate is positioned midway between these two landmarks, typically about one to one and a half centimeters below the exterior occipital protuberance. This places the rod anchor bolts several centimeters or more lower than occurs with the T-plate positioned as shown in Figure 12D. When installed in this position, the anchor bolts are more dependably couched in a thicker bed of soft tissue, facilitating surgical wound closure, particularly for wasted or elderly patients having very little or very inelastic skin and soft tissue over the skull.

This completes a description of a number of representative embodiments of the various anchor plates and the anchor bolt structures for orthopedic fixation in accordance with the present invention. As indicated above, one of ordinary skill in the art will readily appreciate that similarly shaped plates may be used as well. For example, plates characterized as having a V-, U-, or rotated C- shape may be used as well to provide lateral offset positions for rod-anchoring bolts in appropriate positions vertically offset from the occipital protuberance.

While not specifically illustrated, the aperture 5 in the plate may be a circular hole, or an elongated hole or may be an open-ended or a closed-ended slot. In the latter three cases, the bolt 12, in addition to being fully rotatable about its axis, may slide to an arbitrary linear position along the slot before it is tightened in position. The upper surface 13a of the base of the bolt (Figure 1), as well as the lower surface 24a of the support platform (Figure 3), may be toothed, knurled, roughened or otherwise textured to assure that these surfaces grip the plate when tightened and prevent the bolt from rotating or shifting position. Alternatively, or in addition the corresponding contact region of the plate may have such a gripping texture or surface finish. Furthermore, the plate component may have plural openings, grooves or channels adapted to receive one or more bolt/collar assemblies of the invention, and may have several grooves extending along different directions to provide a range of position options. The bolt and support plate may also be secured to each other by detents or interference fit, in variations of the configurations illustrated in the figures.

The occipital plate may be used with fixation rods that may have either a fixed diameter, or may have different diameters at different portions along their length (e.g., transition rods with multiple or stepped-diameter, transitioning from a four millimeter diameter at the thoracic vertebrae to three millimeter diameter in the cervical/cranial region). To fix the upper spine and relieve compression or stabilize the vertebrae for fusion, systems of the invention may also employ one or more cables (e.g., sublaminar cables to support the cervical vertebrae), which are applied between the rods to secure the transition rods to the spine.

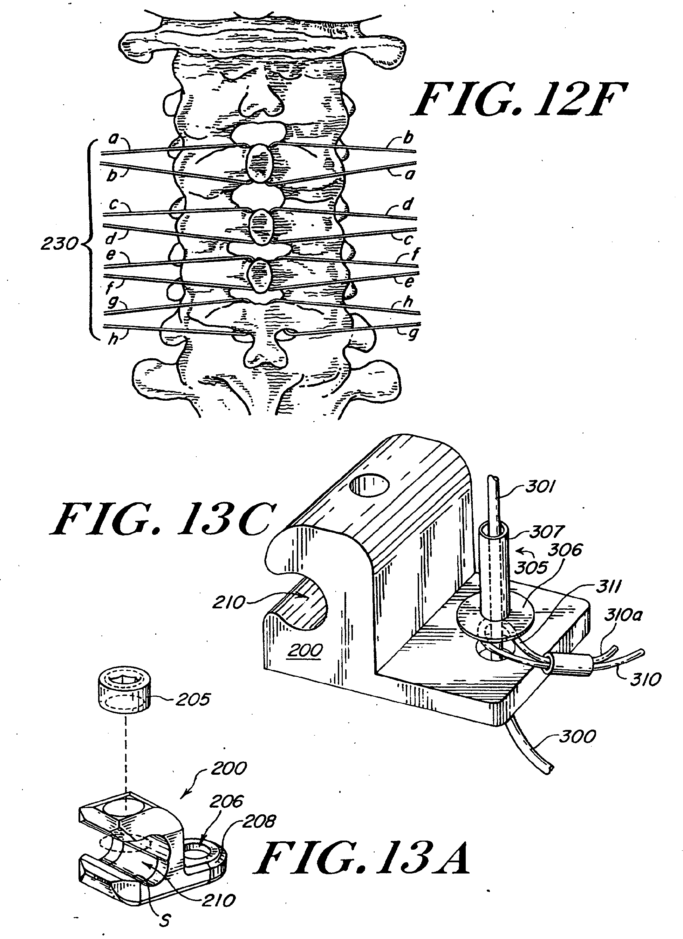

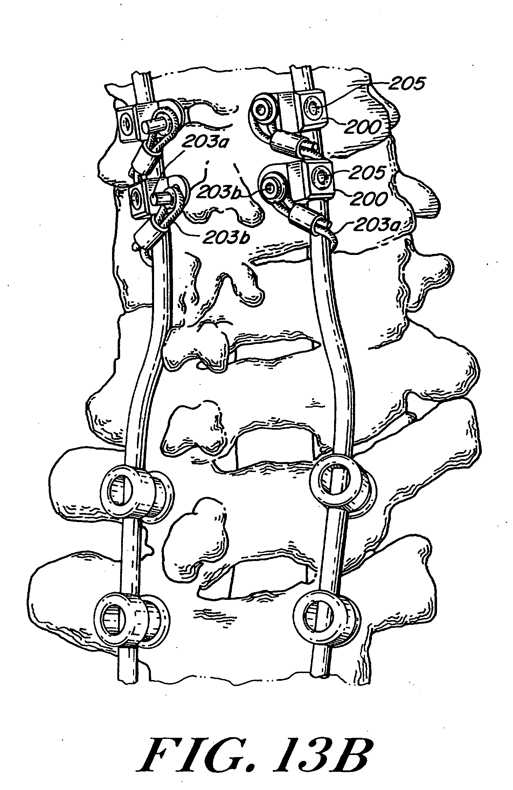

The applicant provides a new cabling system which can be used together with the present invention, with a cable connector mechanism 200 as shown in Figure 13A configured to clamp onto a fixation rod and form a rigid, but selectively positioned, cable anchor. The cable connector 200 has a channel 210 sized to fit onto the fixation rod 3 (Figures 1, 13B), and a set screw 205 that tightens down to clamp against the rod 3 and secure the connector 200 in a desired, fixed position on the rod. As illustrated, the channel 210 is open on one side, and its contour is rounded such that the rod seats low against the contour of the floor of the channel. As a result, when the set screw 205 is tightened, the rising wall or step region S of the body prevents any lateral motion of the rod. The cable connector 200 also has a lateral flange 208 extending out from the channeled body, with an eyelet, or aperture or bolt hole 206 through which a cable is secured. The aperture 206 thus provides a fixed point for vertebra-to-rod cabling.

With this construction, an occipital plate, such as a T-, Y- or inverted Y-plate 102a or 102b is installed, several vertebra anchor screws are placed, and the rods 3 are contoured and fitted into position between the slotted anchors of the occipital plate and the anchor screws attached to the vertebrae. In addition, one or more cable connectors 200 are fitted onto the rod and cables are laced to one or more vertebrae, tensioned and fastened to the connectors to firmly secure the rod to the cervical spine, binding and supporting specific vertebrae. The connectors 200 provide fixed anchor points for the cables at the level of the vertebra, resulting in more secure fixation. In particular, they provide fastening such that the cable has relatively short spans that do not allow a supported vertebra to shift appreciably, and the cable itself is not free to shift vertically. Figure 12F illustrates sublaminar threading of Songer cables, which is preferably carried out at each of the vertebrae to be supported, before installation of the rods 3a,3b, as described further below.

The fixation system may be installed as follows. When cabling is to be used for fixation of the cervical (or other) vertebrae, the cables may initially be inserted at all levels to be fused. Advantageously, by using titanium double cables with a leader, two cables may be applied simultaneously at each vertebral level using one sublaminar passage. For Songer cables, the procedure may be effected by contouring the double cable leader in a C-shape conforming to the specific anatomy. Starting caudally, the contoured leader is introduced inferiorly, beneath and around the relevant lamina. As it emerges on the superior side, the leader is caught with rubber-clad forceps or a blunt hook and pulled upward to maintain tension on the cable. This minimizes contact with the underlying dura. The leader is then cut and the cables are separated laterally and clamped at each side of the wound using rubber-clad forceps. The process is repeated sequentially at all levels to be fused, resulting in a set of cable segments 230 as shown in Figure 12F . The segments are labeled (a) - (h) to more clearly depict the left and right sides of each cable.

The two rods 3a, 3b are then contoured and are cut, if necessary, to assure a precise anatomical fit, and their upper and lower ends are captured in the anchor bolts of the occipital plate and the vertebral anchor screws, respectively, bilaterally. Final tightening of the slotted anchor bolts is performed later, after the cables are securely in place.

Once the rods are loosely secured, the inferior cable ends at each level are placed to the outside of each rod. Beginning with the most distal cable, the leadered end is passed through the eyelet 206 of a cable connector200, and through a cable eyelet (formed by crimping ferrule about a looped end of a cable) and finally draped over the side. The connector 200 is then placed on the rod 3a or 3b, and loosely secured by tightening the set screw 205. This procedure is repeated, providing one cable connector for each cable; the connector is loosely secured to one of the rods at each level. The cables are then tensioned and crimped, and the cable connectors 200 are each tightened down onto the rods. Final tightening of the fixation rods in their anchor bolts may then be effected, Figure 13b illustrates a model spine with two cervical vertebrae cabled in this manner.

Figure 13C is an enlarged line drawing illustrating details of cable attachment to cable connector 200. As shown, a cable 300 has its leader end 301 inserted through the eyelet 206 of a cable connector. A cable eyelet or end-loop 311 of a different cable 310 (or the other end of the same cable according to some methods) is passed over the leader 301 and nested down against the surface of the connector 200, and a top-hat crimp termination 305 is used to secure the leader end. The crimp termination 305 has a brim 306 that is of greater diameter than the loop opening of cable eyelet 311, and also greater than the connector eyelet 206. The crimp termination also has a cylindrical body 307 through which the leader has been fitted, and this is crimped, or loosely set; once the two ends 301, 311 have been positioned at the connector 200, so that both cable ends are captured at the connector. As described above, the cable connector assembly is fastened to one of the fixation rods 3a, 3b during a multi-step procedure in which the cables then rods, then cable connectors are positioned. A tensioning tool (not shown) then tensions each cable by pulling the leader 301 (e.g., gripping the leader and pushing against the termination) while crimping the body portion 307 of the top hat termination securely on the cable leader end 301 for final tensioning once the cables and other components have been aligned, attached and undergone preliminary tightening. The loops 311 may be preformed (e.g., the cables as shown in Figure 12F may be shipped ready to use with one straight and one looped end), or the loops may be formed during the procedure, by crimping a sleeve or ferrule over the cable 310 and its looped-back end 310a to form the cable eyelet 311.

It will be understood that the use of cables to secure the rods to vertebrae has been a generally accepted practice in situations where additional support is necessary without using anchor screws in the affected vertebrae. However, it will be understood that rather than passing a sublaminar cable about the vertebra, it is also possible to place relatively small anchor screws in the cervical vertebrae for directly clamping to the fixation rod.

Anchor plates of the invention have particular utility in an occipital fixation assembly, wherein the plate portion is shaped to firmly seat against the occiput. The ability to conveniently provide a complete rotation of the slotted rod anchor bolt while allowing some translation or offset is of great utility in minimizing misalignment of the transition rod and avoiding the application of unwanted pull-out forces on the anchor screws. However, the constructions of the invention also have utility in diverse other embodiments.

In the various embodiments of occipital plates described herein, the plate itself is configured to anchor to the skull along the midline of the occiput, where bone is thicker and presents a stronger substrate for anchoring, and to secure fixation rods along the branches or arms of the occipital plate adjacent to the anchor screws. Advantageously, the inverted Y-plate embodiment positions the rod-securing slotted bolts or cap assemblies below the nuchal line, where the presence of sufficient or thicker soft tissue ensures that wound closure may be effected without excessive stretching or stress. The invention may alternatively achieve this result with plate shapes such as a V.

The invention being thus disclosed and illustrative embodiments depicted herein, further variations and modifications of the invention, will occur to those skilled in the art.

## Claims

1. An anchor assembly for spinal fixation, comprising
an anchor plate (102, 102a, 102b) configured to be anchored centrally on a skull, the plate (102, 102a, 102b) having a central portion (110), first and second arms (112, 114) extending to sides thereof and a circular hole, an elongated hole, a slot, an aperture or a channel in one of the arms thereof,
at least one bolt (12) adapted a) to be seated in the circular hole, the elongated hole, the slot, the aperture or the channel in the arm thereof and b) to receive a fixation member, the bolt having a slot therein configured to capture and clamp the fixation member (3a, 3b);
**characterised in that**:
the bolt (12), when seated in the arm, is freely rotatable relative to the arm, before clamping, for positioning; and
the anchor plate (102, 102a, 102b) is configured further to be anchored centrally on the skull below the external occipital protuberance (P) by having either a T-shape with a midline notch (N) on its superior aspect for positioning below the external occipital protuberance (P), or a Y-shape, or a V-shape, or a U-shape, or a rotated C-shape.

2. The anchor assembly of claim 1, wherein the plate (102, 102a, 102b) is bent or curved out-of-plane to fit the curved bone surface of the scull.

3. The anchor assembly of claim 1 or claim 2, wherein the plate (102, 102a, 102b) is a Y-shape and it is configured to mount onto the scull in an inverted Y orientation, still below the external occipital protuberance (P).

4. The anchor assembly of any one of the preceding claims, wherein each arm has a circular hole, an elongated hole, a slot, an aperture or a channel and a bolt is seated in each arm.

5. The anchor assembly of any one of the preceding claims, wherein the bolt is seated in one of the arms (112, 114) and it forms a unitary and non-separating assembly with said anchor plate (102b) for one piece installation.

6. The anchor assembly of any one of the preceding claims, wherein the plate (102, 102a, 102b) has an elongated aperture or channels (105a) through which the bolt (12) fits from one side to extend through to the other side, the bolt (12) being adjustably positionable along the length of the elongated aperture or channel (105a) before clamping.

7. The anchor assembly according to claim 6, wherein the aperture or channel (105a) is elongated along a longitudinal axis of said arm (112, 114).

8. The anchor assembly according to any one of the preceding claims, wherein the bolt (12) is affixed to the anchor plate (102b) by a support platform (22) configured to fit about the bolt (12) and having a support surface alignable with the slot (16) of the bolt (12), the bolt (12) and support platform (22) together capturing the plate (102b) therebetween to form a unitary assembly with the bolt (12) being movable in said plate (102b) to define an angular orientation of the slot (16).

9. The anchor assembly of claim 8, wherein said support platform (22) includes a sleeve portion (24b) that fits into an aperture (105a) in the plate (102b).

10. The anchor assembly of claim 9, wherein said support platform sleeve portion (24b) extends only partially through said plate (1 02b).

11. The anchor assembly of claim 8, claim 9 or claim 10, wherein the bolt (12) has a base (13) with an upper surface (13a) and at least one of said upper surface (13a) and a lower surface (24a) of the support platform (22) is textured for preventing movement against the plate (102b) when the bolt (12) is under tension.

12. The anchor assembly of any one of claims 8 to 11, wherein said support platform is a split washer (142, 152) having a seating surface for supporting the fixation member, and a catch or detent (143, 152a) for capturing the bolt (140, 150).

13. The anchor assembly of any one of claims 8 to 11, wherein said support platform (22) is a washer or collar-like support element having a seating surface for supporting the fixation member, the platform (22) having a pair of radially directed holes (25) that extend inward from a circumferentially outer wall of the platform (22) to a depth close to a radially inner surface to serve as access holes to permit swaging of the remaining thin-walled inner collar portion of the support platform (22) to the bolt while the plate (2) is captured between the bolt's base and the platform.

14. The anchor assembly of any one of claims 8 to 11, wherein the bolt (120) is formed with a protruding circumferential ridge (121) positioned to capture the support platform (122) and the support platform (122) is a washer or collar-like support element having a seating surface for supporting the fixation member, the platform (122) having a corresponding groove or recess (123) into which the ridge fits to lock the platform (122) onto the bolt (120).

15. The anchor assembly of any one of claims 8 to 11, wherein the bolt (120) possesses a recessed circumferential groove (131) and the support platform (122) is a washer or collar-like support element having a seating surface for supporting the fixation member, the platform (122) having a corresponding portion with a protruding ridge (136) that snaps into the groove (131) to retain the two parts together.

16. The anchor assembly of any one of the preceding claims, wherein the plate (102. 102a, 102b) has a stepped slot (105a) in which the bolt (12) is seated and along which the bolt (12) slides to a position aligned for receiving a fixation rod (3a, 3b).

17. The anchor assembly of any one of the preceding claims, wherein the plate's central portion (110) has at least one anchor screw hole disposed therein.

18. A spinal fixation system comprising:
an anchor assembly according to any one of the preceding claims,
a fixation rod (3a, 3b) having a proximal end portion for clamping in the slot (16) of one of the rod-clamping anchor structures and a distal end portion for anchoring to a vertebra; and
at least one cable connector (200) fastened to the fixation rod (3a, 3b) at a selected position between said proximal and distal end portions, said cable connector (200) including an eyelet (206) defining a connection point to secure a spinal fixation cable (300, 310) at said selected position.

19. The spinal fixation system of claim 18, wherein:
the cable connector (200) comprises a body having a generally elongated channel (210) extending therethrough sized to receive the fixation rod therein, said channel (210) being open sided such that the body may be placed from a side onto the fixation rod (3a, 3b) at a selected position while the rod (3a, 3b) is at least partly anchored in position;
the body further having a clamping screw (205) fitted in said body adjacent said channel (210) such that the clamping screw (205) locks the fixation rod (3a, 3b), securely fixing the position of the cable connector (200) on the rod (3a, 3b), whereby the eyelet (206) will secure a cable (300, 310) at said selected position.

## Patentansprüche

1. Ankeranordnung für die spinale Fixierung, mit
einer Ankerplatte (102, 102a, 102b), die so gestaltet ist, dass sie zentral auf einem Schädel verankert werden kann, wobei die Platte (102, 102a, 102b) einen mittleren Bereich (110), einen ersten und einen zweiten Arm (112, 114), die sich zu dessen Seiten erstrecken, und ein kreisförmiges Loch, ein längliches Loch, einen Schlitz, eine Öffnung oder einen Kanal in einem ihrer Arme hat,
wenigstens einem Bolzen (12), der dazu ausgelegt ist, a) in dem kreisförmigen Loch, dem länglichen Loch, dem Schlitz, der Öffnung oder dem Kanal in dem Arm zu sitzen und b) ein Fixierelement aufzunehmen, wobei der Bolzen einen Schlitz enthält, der so gestaltet ist, dass er das Fixierelement (3a, 3b) hält und verklemmt;
**dadurch gekennzeichnet, dass**
der Bolzen (12), wenn er in dem Arm sitzt, in Bezug auf den Arm vor dem Verklemmen zum Positionieren frei drehbar ist; und
die Ankerplatte (102, 102a, 102b) so gestaltet ist, dass sie weiter zentral auf dem Schädel unterhalb der Protuberantia occipitalis externa (P) verankert werden kann, indem sie entweder eine T-Form mit einer mittigen Kerbe (N) auf ihrer höheren Seite zum Positionieren unterhalb de Protuberantia occipitalis extema (P) oder eine Y-Form oder eine V-Form oder eine U-Form oder eine gedrehte C-Form hat.

2. Ankeranordnung nach Anspruch 1, bei der die Platte (102, 102a, 102b) aus der Ebene gebogen oder gekrümmt ist, um an die gekrümmte Knochenoberfläche des Schädels angepasst zu sein.

3. Ankeranordnung nach Anspruch 1 oder Anspruch 2, bei der die Platte (102, 102a, 102b) eine Y-Form hat und sie so gestaltet ist, dass sie in einer umgekehrten Y-Ausrichtung auf dem Schädel angeordnet wird, immer noch unterhalb der Protuberantia occipitalis extema (P).

4. Ankeranordnung nach einem der vorangehenden Ansprüche, bei der jeder Arm ein kreisförmiges Loch, ein längliches Loch, einen Schlitz, eine Öffnung oder einen Kanal hat und ein Bolzen in jeden Arm eingesetzt ist.

5. Ankeranordnung nach einem der vorangehenden Ansprüche, bei der der Bolzen in einen der Arme (112, 114) eingesetzt ist und er eine einheitliche und nicht trennbare Anordnung mit der Ankerplatte (102b) für den einstückigen Einbau bildet.

6. Ankeranordnung nach einem der vorangehenden Ansprüche, bei der die Platte (102, 102a, 102b) eine längliche Öffnung oder einen Kanal (105a) hat, durch den der Bolzen (12) von einer Seite her passt, um sich zu der anderen Seite zu erstrecken, wobei der Bolzen (12) entlang der Länge der länglichen Öffnung oder des Kanals (105a) vor dem Verklemmen einstellbar positionierbar ist.

7. Ankeranordnung nach Anspruch 6, bei der die Öffnung oder der Kanal (105a) entlang einer Längsachse des Arms (112, 114) länglich ausgebildet ist.

8. Ankeranordnung nach einem der vorangehenden Ansprüche, bei der der Bolzen (12) an der Ankerplatte (1 02b) über eine Trägerplattform (22) befestigt ist, die so gestaltet ist, dass sie um den Bolzen (12) passt und eine Trägerfläche hat, die mit dem Schlitz (16) des Bolzens (12) auszurichten ist, wobei der Bolzen (12) und die Trägerplattform (22) zusammen die Platte (102b) zwischen sich halten, um eine einheitliche Anordnung zu bilden, wobei der Bolzen (12) in der Platte (102b) bewegbar ist, um eine winklige Ausrichtung des Schlitzes (16) zu definieren.

9. Ankeranordnung nach Anspruch 8, bei der die Trägerplattform (22) einen Hülsenabschnitt (24b) umfasst, der in eine Öffnung (105a) in der Platte (102b) passt.

10. Ankeranordnung nach Anspruch 9, bei der der Hülsenabschnitt (24b) der Trägerplattform sich nur teilweise durch die Platte (102b) erstreckt.

11. Ankeranordnung nach Anspruch 8, Anspruch 9 oder Anspruch 10, bei der der Bolzen (12) eine Basis (13) mit einer oberen Fläche (13a) hat und bei der wenigstens eine aus der oberen Fläche (13a) und einer unteren Fläche (24a) der Trägerplattform (22) texturiert ist, um eine Bewegung gegen die Platte (102b) zu verhindern, wenn der Bolzen (12) unter Spannung steht.

12. Ankeranordnung nach einem der Ansprüche 8 bis 11, bei der die Trägerplattform eine geteilte Scheibe (142, 152) mit einer Sitzfläche zum Halten des Fixierelementes und einer Falle oder Vertiefung (143, 152a) zum Halten des Bolzens (140, 150) ist.

13. Ankeranordnung nach einem der Ansprüche 8 bis 11, bei der die Trägerplattform (22) ein scheiben- oder kragenartiges Trägerelement ist, das eine Sitzfläche zum Halten des Fixierelementes hat, wobei die Plattform (22) ein Paar radial ausgerichteter Löcher (25) hat, die sich von einer Umfangsaußenwand der Plattform (22) nach Innen zu einer Tiefe nahe einer radial inneren Fläche erstrecken, um als Zugangslöcher zu dienen, um das Schmieden des verbleibenden dünnwandigen inneren Kragenabschnittes der Trägerplattform (22) an den Bolzen zu erlauben, während die Platte (2) zwischen der Basis des Bolzens und der Plattform gehalten wird.

14. Ankeranordnung nach einem der Ansprüche 8 bis 11, bei der der Bolzen (120) mit einer hervorstehenden Umfangsrippe (121) gebildet ist, die so angeordnet ist, dass sie die Trägerplattform (122) hält, und die Trägerplattform (122) ein scheiben- oder kragenartiges Trägerelement mit einer Sitzfläche zum Halten des Fixierelementes ist, wobei die Plattform (122) eine entsprechende Nut oder Ausnehmung (123) hat, in die die Rippe passt, um die Plattform (122) auf dem Bolzen (120) zu verriegeln.

15. Ankeranordnung nach einem der Ansprüche 8 bis 11, bei der der Bolzen (120) eine ausgenommene Umfangsnut (131) besitzt und die Trägerplattform (122) ein scheiben- oder kragenartiges Trägerelement mit einer Sitzfläche zum Halten des Fixierelementes ist, wobei die Plattform (122) einen entsprechenden Bereich mit einer hervorstehenden Rippe (136) hat, die in die Nut (131) einrastet, um die beiden Teile zusammenzuhalten.

16. Ankeranordnung nach einem der vorangehenden Ansprüche, bei der die Platte (102, 102a, 102b) einen abgestuften Schlitz (105a) hat, in dem der Bolzen (12) sitzt und entlang dem der Bolzen (12) in eine Position gleitet, die für das Aufnehmen einer Fixierstange (3a, 3b) ausgerichtet ist.

17. Ankeranordnung nach einem der vorangehenden Ansprüche, bei der der Mittelbereich (110) der Platte wenigstens ein Ankerschraubenloch hat, das darin angeordnet ist.

18. Spinales Fixiersystem, das aufweist:
eine Ankeranordnung nach einem der vorangehenden Ansprüche;
eine Fixierstange (3a, 3b) mit einem proximalen Endbereich zum Einklemmen in den Schlitz (16) einer der die Stange verklemmenden Ankerstrukturen und mit einem distalen Endbereich zum Verankern mit einem Wirbel; und
wenigstens einen Kabelverbinder (200), der an der Fixierstange (3a, 3b) an einer ausgewählten Position zwischen dem proximalen und dem distalen Endbereich befestigt ist,
wobei der Kabelverbinder (200) eine Öse (206) umfasst, die einen Verbindungspunkt definiert, um ein spinales Fixierkabel (300, 310) an der ausgewählten Position zu sichern.

19. Spinales Fixiersystem nach Anspruch 18, bei dem:
der Kabelverbinder (200) einen Körper mit einem im Allgemeinen länglichen Kanal (210), der sich durch diesen erstreckt, aufweist, der so bemessen ist, dass er die Fixierstange aufnimmt, wobei der Kanal (210) offenseitig ist, so dass der Körper von einer Seite auf der Fixierstange (3a, 3b) in eine ausgewählte Position gebracht werden kann; während die Stange (3a, 3b) wenigstens teilweise in ihrer Position verankert ist;
wobei der Körper weiter eine Klemmschraube (205) hat, die in den Körper benachbart dem Kanal (210) derart eingepasst ist, dass die Klemmschraube (205) die Fixierstange (3a, 3b) verriegelt, wobei die Position des Kabelverbinders (200) auf der Stange (3a, 3b) sicher festgelegt ist, wodurch die Öse (206) ein Kabel (300, 310) in der ausgewählten Position sichern wird.

## Revendications

1. Ensemble d'ancrage pour fixation spinale, comprenant :
- une plaque d'ancrage (102, 102a, 102b) configurée pour être ancrée de manière centrale sur un crâne, la plaque (102, 102a, 102b) ayant une portion centrale (110), des premier et deuxième bras (112, 114) s'étendant sur les côtés de celle-ci et un trou circulaire, un trou allongé, une fente, une ouverture ou un canal dans l'un des bras de celle-ci,
- au moins un boulon (12) apte a) à être assis dans le trou circulaire, le trou allongé, la fente, l'ouverture ou le canal dans le bras de celle-ci et b) à recevoir un membre de fixation, le boulon ayant une fente configurée à l'intérieur de celui-ci pour capturer et serrer le membre de fixation (3a, 3b) ;
**caractérisé en ce que** :
- le boulon (12), lorsqu'il est assis dans le bras, peut tourner librement par rapport au bras, avant d'être serré, pour être positionné ; et
- la plaque d'ancrage (102, 102a, 102b) est en outre configurée pour être ancrée de manière centrale sur le crâne au-dessous de la protubérance occipitale externe (P) en ayant une forme en T avec une encoche de ligne médiane (N) sur son aspect supérieur pour être positionnée au-dessous de la protubérance occipitale externe (P), ou une forme en Y, ou une forme en V, ou une forme en U ou une forme en C retourné.

2. Ensemble d'ancrage selon la revendication 1, dans lequel la plaque (102, 102a, 102b) est pliée ou incurvée hors plan pour correspondre à la surface osseuse incurvée du crâne.

3. Ensemble d'ancrage selon la revendication 1 ou 2, dans lequel la plaque (102, 102a, 102b) a une forme en Y et est configurée pour être montée sur le crâne dans une orientation de Y inversé, toujours au-dessous de la protubérance occipitale externe (P).

4. Ensemble d'ancrage selon l'une quelconque des revendications précédentes, dans lequel chaque bras a un trou circulaire, un trou allongé, une fente, une ouverture ou un canal et un boulon est assis dans chaque bras.

5. Ensemble d'ancrage selon l'une quelconque des revendications précédentes, dans lequel le boulon est assis dans l'un des bras (112, 114) et forme un ensemble unitaire non séparé avec ladite plaque d'ancrage (102b) pour une installation d'un seul tenant.

6. Ensemble d'ancrage selon l'une quelconque des revendications précédentes, dans lequel la plaque (102, 102a, 102b) a une ouverture ou un canal allongé (105a) à travers lequel le boulon (12) entre d'un côté pour s'étendre jusqu'à l'autre côté, le boulon (12) étant positionnable de manière ajustable le long de la longueur de l'ouverture ou du canal allongé (105a) avant d'être serré.

7. Ensemble d'ancrage selon la revendication 6, dans lequel l'ouverture ou le canal (105a) est allongé le long d'un axe longitudinal dudit bras (112, 114).

8. Ensemble d'ancrage selon l'une quelconque des revendications précédentes, dans lequel le boulon (12) est fixé sur la plaque d'ancrage (102b) par une plate-forme de support (22) configurée pour s'adapter autour du boulon (12) et ayant une surface de support pouvant être alignée avec la fente (16) du boulon (12), le boulon (12) et la plate-forme de support (22) capturant ensemble la plaque (102b) entre eux pour former un ensemble unitaire avec le boulon (12) qui peut être déplacé dans ladite plaque (102b) pour définir une orientation angulaire de la fente (16).

9. Ensemble d'ancrage selon la revendication 8, dans lequel ladite plate-forme de support (22) comprend une portion de douille (24b) qui entre dans une ouverture (105a) dans la plaque (102b).

10. Ensemble d'ancrage selon la revendication 9, dans lequel ladite portion de douille de plate-forme de support (24b) s'étend uniquement partiellement à travers ladite plaque (102b).

11. Ensemble d'ancrage selon la revendication 8, la revendication 9 ou la revendication 10, dans lequel le boulon (12) a une base (13) avec une surface supérieure (13a) et au moins l'une de ladite surface supérieure (13a) et d'une surface inférieure (24a) de la plate-forme de support (22) est texturée pour empêcher un mouvement contre la plaque (102b) lorsque le boulon (12) est sous tension.

12. Ensemble d'ancrage selon l'une quelconque des revendications 8 à 11, dans lequel ladite plate-forme de support est une rondelle fendue (142, 152) ayant une surface d'assise pour supporter le membre de fixation, et un loquet ou une détente (143, 152a) pour capturer le boulon (140, 150).

13. Ensemble d'ancrage selon l'une quelconque des revendications 8 à 11, dans lequel ladite plate-forme de support (22) est une rondelle ou un élément de support en forme de collier ayant une surface d'assise pour supporter le membre de fixation, la plate-forme (22) ayant une paire de trous dirigés radialement (25) qui s'étendent vers l'intérieur à partir d'une paroi extérieure circonférentiellement de la plate-forme (22) à une profondeur proche d'une surface intérieure radialement pour servir de trous d'accès pour permettre le balancement de la portion de collier intérieur de paroi fine restante de la plate-forme de support (22) vers le boulon pendant que la plaque (2) est capturée entre la base du boulon et la plate-forme.

14. Ensemble d'ancrage selon l'une quelconque des revendications 8 à 11, dans lequel le boulon (120) est formé avec une arête circonférentielle protubérante (121) positionnée pour capturer la plate-forme de support (122) et la plate-forme de support (122) est une rondelle ou un élément de support en forme de collier ayant une surface d'assise pour supporter le membre de fixation, la plate-forme (122) ayant une gorge ou un renfoncement correspondant (123) dans lequel entre l'arête pour verrouiller la plate-forme (122) sur le boulon (120).

15. Ensemble d'ancrage selon l'une quelconque des revendications 8 à 11, dans lequel le boulon (120) possède une gorge circonférentielle renfoncée (131) et la plate-forme de support (122) est une rondelle ou un élément de support en forme de collier ayant une surface d'assise pour supporter le membre de fixation, la plate-forme (122) ayant une portion correspondante avec une arête protubérante (136) qui se ferme avec un bruit sec dans la gorge (131) pour retenir les deux parties ensemble.

16. Ensemble d'ancrage selon l'une quelconque des revendications précédentes, dans lequel la plaque (102, 102a, 102b) a une fente en escalier (105a) dans laquelle le boulon (12) est assis et le long de laquelle le boulon (12) coulisse jusqu'à une position alignée pour recevoir une tige de fixation (3a, 3b).

17. Ensemble d'ancrage selon l'une quelconque des revendications précédentes, dans lequel la portion centrale (110) de la plaque a au moins un trou de vis d'ancrage disposé à l'intérieur de celle-ci.

18. Système de fixation spinale comprenant :
■ un ensemble d'ancrage selon l'une quelconque des revendications précédentes ;
■ une tige de fixation (3a, 3b) ayant une portion d'extrémité proximale pour le serrage dans la fente (16) de l'une des structures d'ancrage de serrage de tige et une portion d'extrémité distale pour l'ancrage sur une vertèbre ; et
■ au moins un connecteur de câble (200) attaché à la tige de fixation (3a, 3b) à une position sélectionnée entre lesdites portions d'extrémité proximale et distale, ledit connecteur de câble (200) comprenant un oeillet (206) définissant un point de connexion pour fixer un câble de fixation spinale (300, 310) à ladite position sélectionnée.

19. Système de fixation spinale selon la revendication 18, dans lequel :
■ le connecteur de câble (200) comprend un corps ayant un canal généralement allongé (210) s'étendant à travers celui-ci dimensionné pour recevoir la tige de fixation à l'intérieur de celui-ci, ledit canal (210) étant de côté ouvert de sorte que le corps puisse être placé d'un côté sur la tige de fixation (3a, 3b) à une position sélectionnée pendant que la tige (3a, 3b) est au moins partiellement ancrée en position ;
■ le corps ayant en outre une vis de serrage (205) entrée dans ledit corps adjacent audit canal (210) de sorte que la vis de serrage (205) verrouille la tige de fixation (3a, 3b), en fixant de manière sécurisée la position du connecteur de câble (200) sur la tige (3a, 3b), de telle manière que l'oeillet (206) fixe un câble (300, 310) à ladite position sélectionnée.
